(19) Europäisches Patentamt
European Patent Office
Office européen des brevets

(11) EP 4 186 902 A1

(12) **EUROPEAN PATENT APPLICATION**
published in accordance with Art. 153(4) EPC

(43) Date of publication:
**31.05.2023 Bulletin 2023/22**

(21) Application number: **21846285.1**

(22) Date of filing: **22.07.2021**

(51) International Patent Classification (IPC):
***C07D 471/04*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 471/04**

(86) International application number:
**PCT/CN2021/107815**

(87) International publication number:
**WO 2022/017449 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.07.2020 CN 202010711260**

(71) Applicant: **Nanjing Chia Tai Tianqing Pharmaceutical Co., Ltd.**
**Jiangsu 210046 (CN)**

(72) Inventors:
- **MA, Changyou**
  **Nanjing, Jiangsu 210046 (CN)**
- **TIAN, He**
  **Nanjing, Jiangsu 210046 (CN)**
- **ZHAO, Jianliang**
  **Nanjing, Jiangsu 210046 (CN)**
- **CHEN, Donghui**
  **Nanjing, Jiangsu 210046 (CN)**
- **WU, Jian**
  **Nanjing, Jiangsu 210046 (CN)**
- **XU, Dan**
  **Nanjing, Jiangsu 210046 (CN)**
- **ZHU, Chunxia**
  **Nanjing, Jiangsu 210046 (CN)**
- **TIAN, Zhoushan**
  **Nanjing, Jiangsu 210046 (CN)**

(74) Representative: **Elzaburu S.L.P.**
**Edificio Torre de Cristal**
**Paseo de la Castellana 259 C, planta 28**
**28046 Madrid (ES)**

# (54) SALT OF DIHYDROPYRIDO[2,3-D]PYRIMIDINONE DERIVATIVE, PREPARATION METHOD THEREFOR, AND USE THEREOF

(57) The present application discloses a salt of a dihydropyrido[2,3-d]pyrimidinone derivative, a preparation method therefor and the use thereof. The salt is selected from fumarate, methanesulfonate, isethionate, α-naphthalenesulfonate, p-toluenesulfonate, 1,2-ethanedisulfonate, oxalate, maleate, citrate, succinate, L-(+)-tartrate, hippurate, L-ascorbate, L-malate, benzoate, gentisate, a hydrochloride, a sulfate or a phosphate. The salt of the dihydropyrido[2,3-d]pyrimidinone derivative of the present application can be used in the treatment of breast cancer, prostate cancer, or ovarian cancer.

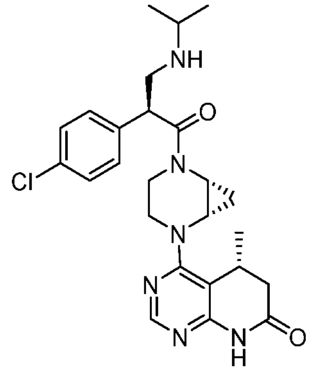

1

EP 4 186 902 A1

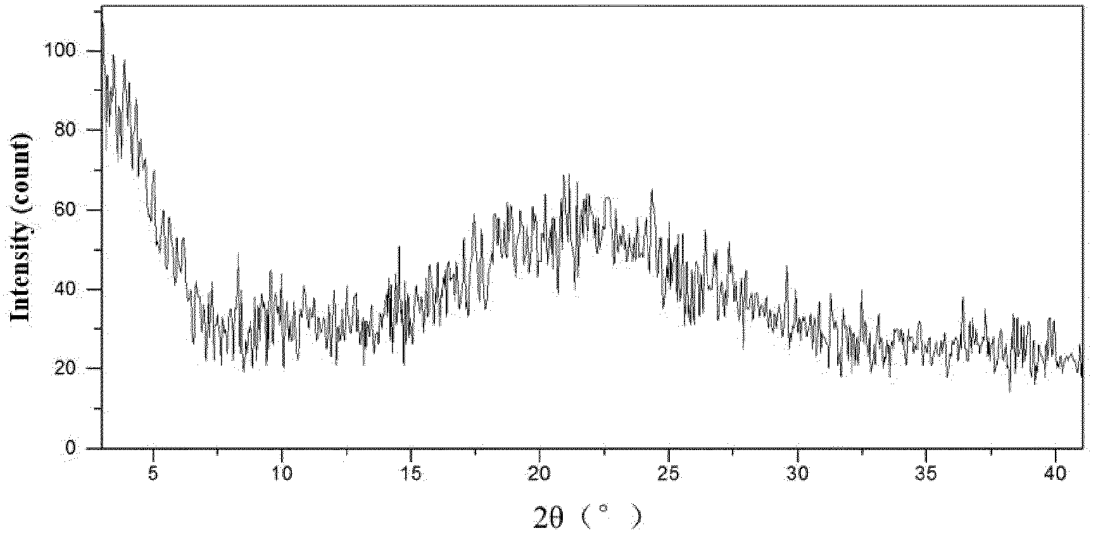


Fig. 10

## Description

### TECHNICAL FIELD

The present application belongs to the field of medicinal chemistry, and specifically relates to a salt of a dihydropyrido[2,3-d]pyrimidinone derivative, a preparation method and medical use thereof.

### BACKGROUND

The PI3K/AKT/mTOR pathway consisting of phosphoinositide-3-kinase (PI3K) and its downstream protein AKT (also known as protein kinase B, PKB), and mammalian target of Rapamycin (mTOR) as a very important intracellular signal transduction pathway, the pathway exerts an extremely important biological function in the process of cell growth, survival, proliferation, apoptosis, angiogenesis, autophagy, etc. Abnormal activation of the pathway will cause a series of diseases such as cancer, neuropathy, autoimmune disease, and hemolymphatic system disease.

AKT is a type of serine/threonine kinase and affects the survival, growth, metabolism, proliferation, migration, and differentiation of cell through numerous downstream effectors. Overactivation of AKT has been observed in more than 50% of human tumors, especially in prostate cancer, pancreatic cancer, bladder cancer, ovarian cancer, and breast cancer. Overactivation of AKT may lead to the formation, metastasis, and drug resistance of tumor. AKT has three isoforms: AKT1, AKT2, and AKT3. As a typical protein kinase, each isoform consists of an amino-terminal pleckstrin homology (PH) domain, a middle ATP-binding kinase domain, and a carboxyl-terminal regulatory domain. About 80% amino acid sequences of the three isoforms are homologous, and only the amino acid sequences in a binding domain between the PH domain and the kinase domain changes greatly.

The current drugs targeting the PI3K/AKT/mTOR signaling pathway mainly include PI3K inhibitors and mTOR inhibitors, while AKT is at the core of the signal transduction pathway. Inhibition of the AKT activity can not only avoid the severe side effects caused by inhibition of upstream PI3K, but also avoid the negative feedback mechanism caused by inhibition of downstream mTOR from affecting the efficacy of a drug. For example, CN101631778A discloses a class of cyclopenta[D]pyrimidine derivatives, CN101578273A discloses a class of hydroxylated and methoxylated cyclopenta[D]pyrimidine derivatives, CN101511842A discloses a class of dihydrofuropyrimidine derivatives, CN101970415A discloses a class of 5H-cyclopenta[d]pyrimidine derivatives, and these compounds inhibit AKT1 with $IC_{50}$ less than 10 μM. However, development of effective and selective AKT inhibitors is still an important direction for current development of tumor-targeting drugs.

### SUMMARY OF THE INVENTION

In one aspect, the present application provides a pharmaceutically acceptable salt of a compound represented by formula 1, which is selected from a salt of organic acid or a salt of inorganic acid. The salt of organic acid is selected from a fumarate, a mesylate, an isethionate, an α-naphthalenesulfonate, a p-toluenesulfonate, a 1,2-ethanedisulphonate, an oxalate, a maleate, a citrate, a succinate, an L-(+)-tartrate, a hippurate, an L-ascorbate, an L-malate, a benzoate, or a gentisate, and the salt of inorganic acid is selected from a hydrochloride, a sulfate, and a phosphate, and the compound represented by formula 1 has the following structure:

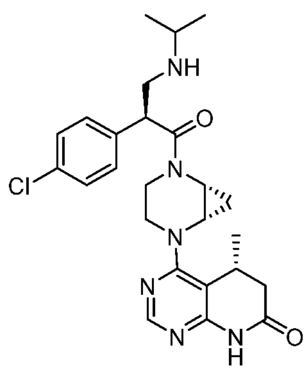

1 .

In some embodiments, the salt of organic acid is a fumarate.

In some embodiments, the salt of inorganic acid is a hydrochloride.

In some embodiments, in the salt of organic acid, a molar ratio of the compound represented by formula 1 to

organic acid is 1: 1.

In some embodiments, in the hydrochloride, a molar ratio of the compound represented by formula 1 to hydrogen chloride is 1: 1 or 1: 2.

In some embodiments, in the hydrochloride, a molar ratio of the compound represented by formula 1 to hydrogen chloride is 1: 2.

In some embodiments, in the sulfate, a molar ratio of the compound represented by formula 1 to sulfuric acid is 1: 1.

In some embodiments, in the phosphate, a molar ratio of the compound represented by formula 1 to phosphoric acid is 1: 1.

It can be understood that the salt of the present application results from a salification reaction of the compound represented by formula 1 with a corresponding acid. In the reaction, the compound represented by formula 1 is converted into cations that bind to acid radicals of the corresponding acid to form the salt. Therefore, in the present application, a molar ratio of the compound represented by formula 1 to an acid can be understood as a molar ratio of cations of the compound represented by formula 1 to acid radicals of the corresponding acid.

In some typical embodiments, the present application provides a fumarate of the compound represented by formula 1, and a molar ratio of the compound represented by formula 1 to fumaric acid is 1: 1, or a molar ratio of cations of the compound represented by formula 1 to acid radicals of fumaric acid is 1: 1.

In some typical embodiments, the present application provides a hydrochloride of the compound represented by formula 1, a molar ratio of the compound represented by formula 1 to hydrogen chloride is 1: 1, or a molar ratio of cations of the compound represented by formula 1 to chloridion is 1: 1, and in this case, the hydrochloride is a monohydrochloride of the compound represented by formula 1.

In some typical embodiments, the present application provides a hydrochloride of the compound represented by formula 1, a molar ratio of the compound represented by formula 1 to hydrogen chloride is 1: 2, or a molar ratio of cations of the compound represented by formula 1 to chloride ions is 1: 2, and in this case, the hydrochloride is a dihydrochloride of the compound represented by formula 1.

In another aspect, the present application provides a preparation method of the pharmaceutically acceptable salt of the compound represented by formula 1, which comprising a step of salification reaction of the compound represented by formula 1 with the corresponding acid.

In some embodiments, a solvent for salification reaction is selected from a mixed solvent of an alcohol solvent and an alkane solvent, a mixed solvent of a ketone solvent and an alkane solvent, a mixed solvent of an ester solvent and an alkane solvent, a mixed solvent of a benzene solvent and an alkane solvent, and a mixed solvent of a halogenated hydrocarbon solvent and an alkane solvent.

In some embodiments, the alcohol solvent is selected from methanol, ethanol or isopropanol, and preferably isopropanol; the ketone solvent is selected from acetone or butanone, and preferably acetone; the ester solvent is selected from ethyl acetate or butyl acetate, and preferably ethyl acetate; the benzene solvent is toluene; the halogenated hydrocarbon solvent is dichloromethane; and the alkane solvent is n-heptane.

In some typical embodiments, the present application provides a preparation method of a fumarate of the compound represented by formula 1, which comprising a step of salification reaction of the compound represented by formula 1 with fumaric acid, and preferably, the solvent for the salification reaction is a mixed solvent of isopropanol and n-heptane.

In some typical embodiments, the present application provides a preparation method of a hydrochloride of the compound represented by formula 1, which comprising a step of salification reaction of the compound represented by formula 1 with hydrochloric acid, and preferably, the solvent for the salification reaction is selected from a mixed solvent of toluene and n-heptane and a mixed solution of ethyl acetate and n-heptane.

In another aspect, the present application also provides a pharmaceutical composition comprising the pharmaceutically acceptable salt of the compound represented by formula 1.

In some embodiments, the pharmaceutical composition further comprises one or more pharmaceutically acceptable carriers.

In some embodiments, the pharmaceutical composition is a solid preparation suitable for oral administration, and preferably tablets or capsules.

In another aspect, the present application also provides the pharmaceutically acceptable salt of the compound represented by formula 1 or a pharmaceutical composition thereof that is used as a medicament.

In another aspect, the present application also provides use of the pharmaceutically acceptable salt of the compound represented by formula 1 or a pharmaceutical composition thereof in the preparation of a medicament for preventing and/or treating an AKT protein kinase-mediated disease or disease state.

In another aspect, the present application also provides use of the pharmaceutically acceptable salt of the compound represented by formula 1 or a pharmaceutical composition thereof in the prevention and/or treatment of an AKT protein kinase-mediated disease or disease state.

In another aspect, the present application also provides a method for preventing and/or treating an AKT protein kinase-mediated disease or disease state, which comprising a step of administering the pharmaceutically acceptable salt of the compound represented by formula 1 or a pharmaceutical composition thereof of the present application to the subject in need.

In another aspect, the present application also provides the pharmaceutically acceptable salt of the compound represented by formula 1 or a pharmaceutical composition thereof of the present application that is used for preventing and/or treating an AKT protein kinase-mediated disease or disease state.

In some embodiments, the AKT protein kinase-mediated disease or disease state is cancer.

In some typical embodiments, the cancer is breast cancer, prostate cancer or ovarian cancer.

In some typical embodiments, the cancer is prostate cancer.

Relevant definitions

Unless otherwise specified, the following terms used in the description and claims have the following meanings.

The pharmaceutically acceptable salts of the present application also include their hydrate forms. The term "pharmaceutically acceptable carrier" refers to a carrier that has no obvious stimulating effect on the body and will not impair the biological activity and performance of an active compound. Pharmaceutically acceptable carriers include, but are not limited to, any diluent, disintegrant, adhesive, glidant, and wetting agent that have been approved by the National Medical Products Administration for human or animal use.

The term "fumaric acid" refers to trans-butenedioic acid having the following structure:

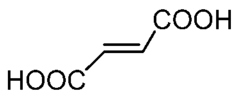

.

The term "alcohol solvent" refers to a derived substance resulting from the substitution of one or more hydrogen atoms on C1-C6 alkane with one or more hydroxyl groups (OH), and the C1-C6 alkane refers to straight-chain or branched-chain alkane containing 1-6 carbon atoms. Specific examples of alcohol solvents include, but are not limited to, methanol, ethanol, isopropanol or n-propanol.

The term "alkane solvent" refers to straight chain or branched or annular alkane containing 5-7 carbon atoms. Specific examples of alkane solvents include, but are not limited to, n-hexane, cyclohexane, n-heptane.

The term "ester solvent" refers to a chain compound containing the ester group -COOR and 3-10 carbon atoms, wherein R is C1-C6 alkyl, and the C1-C6 alkyl refers to straight-chain or branched-chain alkane containing 1-6 carbon atoms. Specific examples of ester solvents include, but are not limited to, methyl acetate, ethyl acetate, and propyl acetate.

The term "halogenated hydrocarbon solvent" refers to a derived substance resulting from the substitution of one or more hydrogen atoms on C1-C6 alkane with one or more halogen atoms, the C1-C6 alkane refers to straight-chain or branched-chain alkane containing 1-6 carbon atoms, and the halogen atom refers to fluorine, chlorine, bromine, iodine. Specific examples of halogenated hydrocarbon solvents include, but are not limited to, dichloromethane and chloroform.

The term "ketone solvent" refers to a chain or ring compound containing the carbonyl group -CO- and 3-10 carbon atoms. Specific examples of ketone solvents include, but are not limited to, acetone, butanone, and cyclohexanone.

The term "benzene solvent" refers to a solvent containing phenyl groups. Specific examples of benzene solvents include, but are not limited to, toluene, xylene, cumene or chlorobenzene.

The term "equivalent" refers to equivalent usage of other raw materials required in accordance with an equivalent relationship of a chemical reaction, taking a basic raw material used at each step as 1 equivalent.

Unless otherwise specified, the abbreviations in the present application have the following meanings:

M: mol/L
mM: mmol/L
nM: nmol/L
Boc: tert-butoxycarbonyl
DCM: dichloromethane
DEA: diethylamine
DIEA: N,N-diisopropylethylamine
HATU: 2-(7-azabenzotriazol)-N,N,N',N'-tetramethyluronium hexafluorophosphate
RT: retention time

SFC: supercritical fluid chromatography
h: hour
min: minute
TK: tyrosine kinase
SEB: fluorescent signal enhancer
HTRF: homogeneous time resolved fluorescence
DTT: dithiothreitol

## BRIEF DESCRIPTION OF THE DRAWINGS

In order to more clearly describe the technical solutions of the examples of the present application and the prior art, the drawings that need to be used in the examples and the prior art will be briefly introduced below. Obviously, the drawings in the following description are some embodiments of the present application only, and those skilled in the art may also obtain other drawings according to these drawings.

Fig. 1 is a schematic diagram of a single molecule of a compound represented by formula 1 of Example 1;
Fig. 2 is a schematic diagram of asymmetric structural unit of an oxalate single crystal of the compound represented by formula 1 of Example 1;
Fig. 3 is an XRPD pattern of a sulfate of the compound represented by formula 1 of Example 2;
Fig. 4 is an XRPD pattern of a phosphate of the compound represented by formula 1 of Example 2;
Fig. 5 is an XRPD pattern of an isethionate of the compound represented by formula 1 of Example 2;
Fig. 6 is an XRPD pattern of an $\alpha$-naphthalenesulfonate of the compound represented by formula 1 of Example 2;
Fig. 7 is an XRPD pattern of an L-malate of the compound represented by formula 1 of Example 2;
Fig. 8 is an XRPD pattern of a monohydrochloride of the compound represented by formula 1 of Example 3;
Fig. 9 is an XRPD pattern of a dihydrochloride of the compound presented by formula 1 of Example 4; and
Fig. 10 is an XRPD pattern of a fumarate of the compound represented by formula 1 of Example 5.

## DETAILED DESCRIPTION OF THE INVENTION

The present application will be described in more detail below with reference to embodiments. However, these specific descriptions are for the purpose of describing the technical solutions of the present application only, and are not intended to limit the present application in any manner.

### Example 1 Preparation of a compound represented by formula 1

**Preparation Example 1** Preparation of intermediate (R)-4-chloro-5-methyl-5,8-dihydropyrido[2,3-d]pyrimidin-7(6H)-one

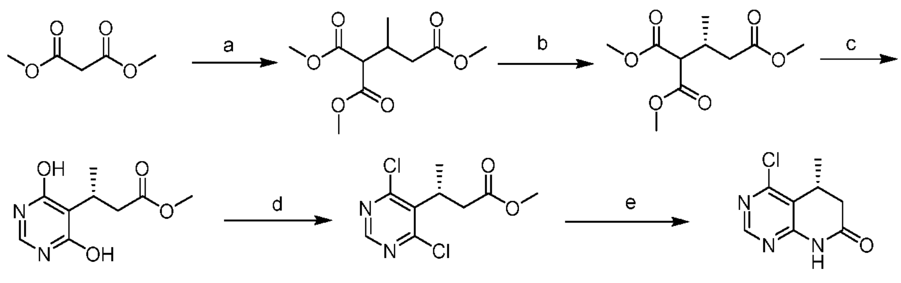


a) Trimethyl 2-methylpropane-1,1,3-tricarboxylate

Under the protection of nitrogen gas, a sodium methylate-methanol solution (30 wt%, 50.32 g) was added to methanol (900 mL), the mixture was heated to 70°C, dimethyl malonate (461.12 g) and ethyl crotonate (349.46 g) were mixed until uniform and dropwise added to the above sodium methylate-methanol solution, and the reaction solution reacted at 70°C for 3 h. After the reaction was completed, the reaction solution was evaporated under reduced pressure to remove the solvent, ethyl acetate (1 L) was added, the mixture was regulated with 4 M hydrochloric acid until the pH of the mixture was 7-8, water (500 mL) was added, and the solution was separated and evaporated under reduced pressure to remove the organic phase so as to yield a yellow liquid (777.68 g). $^1$H NMR (400 MHz, DMSO-$d_6$) $\delta$ (ppm)

3.67 (s, 3H), 3.65 (s, 3H), 3.59 (s, 3H), 3.56 (d, *J*=6.8 Hz, 1H), 2.45-2.58 (m, 2H), 2.23-2.29 (m, 1H), 0.93 (d, *J*=6.8 Hz, 3H).

b) Trimethyl (R)-2-methylpropane-1,1,3-tricarboxylate

Disodium hydrogen phosphate (4.5 g) was dissolved in deionized water (1.5 L) at 25°C, the solution was regulated with 2 N hydrochloric acid until the pH of the solution was 7.05, trimethyl 2-methylpropane-1,1,3-tricarboxylate (150.46 g) and lipase (*Candida rugosa,* 40 g, added in 6 d) were added, the mixture was regulated with a 2 N sodium hydroxide solution until the pH of the mixture was 7.0-7.6, and the reaction solution reacted at 35°C for 6 d. Chirality detection ee%>98%, and chirality detection conditions: Chiralpak IC, 4.6×250 mm, 5 μm, and n-hexane: ethanol=9: 1 (volume ratio). The reaction solution was cooled to 10°C and regulated with 3 M hydrochloric acid until the pH of the reaction solution was 3-4, ethyl acetate (500 mL) was added, the mixture was subjected to suction filtration, an obtained filter cake was washed with ethyl acetate (600 mL), the solution was separated, a saturated sodium bicarbonate aqueous solution (100 mL) was added for washing, the solution was separated, and an obtained organic phase was concentrated to yield a pale-yellow liquid (26.89 g). $^1$H NMR (400 MHz, $CDCl_3$) $\delta$(ppm) 3.74 (s, 6H), 3.68 (s, 3H), 3.46 (d, *J*=7.2 Hz, 1H), 2.71-2.79 (m, 1H), 2.54 (dd, *J*=15.6, 4.8 Hz, 1H), 2.32 (dd, *J*=16.0, 8.4 Hz, 1H), 1.06 (d, *J*=6.8 Hz, 3H).

c) Methyl (R)-3-(4,6-dihydroxypyrimidin-5-yl)butanoate

Under the protection of nitrogen gas, formamidine acetate (11.33 g) was dissolved in methanol (200 mL) at 20°C, the solution was cooled to 0°C, a sodium methylate-methanol solution (30 wt%, 55.62 g) was dropwise added, the reaction solution reacted at 0°C for 60 min, a methanol (60 mL) solution of trimethyl (R)-2-methylpropane-1,1,3-tricarboxylate (24.07 g) was dropwise added, and the reaction solution was naturally heated to 20°C and reacted for 10 h. After the reaction was completed, the reaction solution was cooled to 0°C, regulated with 3 N hydrochloric acid until the pH of the reaction solution was 5-6, evaporated under reduced pressure to remove the solvent, cooled to 0°C, and regulated with 3 N hydrochloric acid until the pH of the reaction solution was 3, after a solid was precipitated, the reaction solution was subjected to suction filtration to collect the solid, and an obtained filter cake was washed with ice water (100 mL) and dried in vacuum to yield a white solid (18.79 g) that was directly used at the next step.

d) Methyl (R)-3-(4,6-dichloropyrimidin-5-yl)butanoate

Under the protection of nitrogen gas, methyl (R)-3-(4,6-dihydroxypyrimidin-5-yl)butanoate (14.63 g) was dispersed into acetonitrile (70 mL) at 22°C, phosphorus oxychloride (26.42 g) and diisopropylethylamine (12.51 g) were dropwise added in sequence, the system released heat obviously and was heated to 60°C, the solids were gradually fully dissolved, and the reaction solution reacted for 18 h. After the reaction was completed, the reaction solution was cooled to 0°C, ethyl acetate (100 mL) was added, the mixture was regulated with a saturated sodium bicarbonate solution until the pH of the mixture was 7-8, extracted with ethyl acetate (50 mL × 3), and evaporated under reduced pressure to remove the organic phase so as to yield a yellow solid (13.89 g) that was directly used at the next step.

e) (R)-4-chloro-5-methyl-5,8-dihydropyrido[2,3-d]pyrimidin-7(6H)-one

Methyl (R)-3-(4,6-dichloropyrimidin-5-yl)butanoate (13.89 g) and ammonia water (25-28 wt%, 70 mL) were placed in a 100 mL high-pressure kettle at 20°C, and the reaction solution was heated to 50°C and reacted for 18 h. After the reaction was completed, the reaction solution was cooled to 0°C and subjected to suction filtration, and an obtained filter cake was beaten with a mixture (30 mL) of petroleum ether and ethyl acetate in a volume ratio of 10: 1 to yield a pale-yellow solid (7.32 g). LC-MS (ESI) m/z: 198 (M+H). $^1$H NMR (300 MHz, $CDCl_3$) $\delta$(ppm) 1.30 (d, J=7.2 Hz, 3H), 2.65-2.69 (m, 1H), 2.86-2.92 (m, 1H), 3.47-3.54 (m, 1H), 8.64 (s, 1H), 10.10 (s, 1H).

**Preparation Example 2** Preparation of (R)-4-((1S,6R)-5-((S)-2-(4-chlorophenyl)-3-(isopropylamino)propionyl)-2,5-diazabicyclo[4.1.0] heptan-2-yl)-5-methyl-5,8-dihydropyrido[2,3-d]pyrimidin-7(6H)-one (compound represented by formula 1)

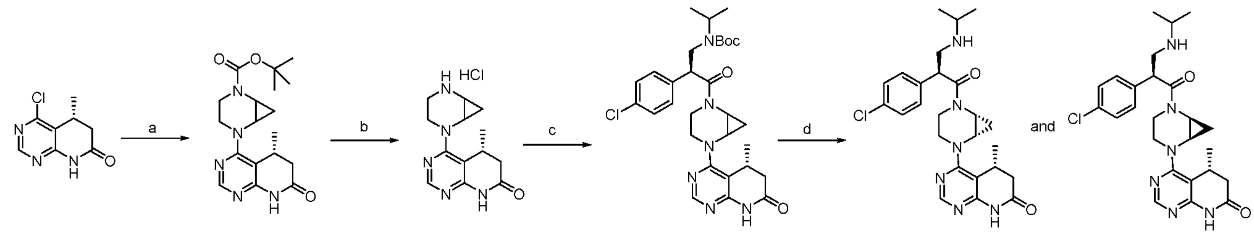


Reaction conditions: a) tert-butyl 2,5-diazabicyclo[4.1.0]heptane-2-carboxylate, N-methylpyrrolidone, and 4-dimethylaminopyridine; b) hydrogen chloride/1,4-dioxane (4.0 M) and dichloromethane; c) (S)-3-((tert-butoxycarbonyl)(isopropyl)amino)-2-(4-chlorophenyl)-propionic acid, 2-(7-benzotriazole oxide)-N,N,N',N'-tetramethyluronium hexafluorophosphate, 4-dimethylaminopyridine, and N,N-dimethylformamide; and d) trifluoroacetic acid and dichloromethane.

a) Tert-butyl 5-((R)-5-methyl-7-oxo-5,6,7,8-tetrahydropyrido[2,3-d]pyrimidin-4-yl)-2,5-diazabicyclo[4.1.0]he ptane-2-carboxylate

Under the protection of nitrogen gas, (R)-4-chloro-5-methyl-5,8-dihydropyrido[2,3-d]pyrimidin-7(6H)-one (0.21 g), tert-butyl 2,5-diazabicyclo[4.1.0]heptane-2-carboxylate (0.31 g), and 4-dimethylaminopyridine (0.39 g) were dissolved in N-methylpyrrolidone (5 mL) at 22°C, and the reaction solution was heated to 140°C and reacted for 3 h. After the reaction was completed, the reaction solution was cooled to 20°C, poured into ice water (20 mL), extracted with ethyl acetate (20 mL × 2), washed with a saturated salt solution (10 mL × 3), evaporated under reduced pressure to remove the solvent, and separated by silica gel column chromatography (petroleum ether: ethyl acetate=(3: 1)-(1: 1)) to yield a pale-yellow liquid (0.28 g). LC-MS (ESI) m/z: 360 (M+H).

b) (SR)-4-(2,5-diazabicyclo [4.1.0]heptan-2-yl)-5-methyl-5, 8-dihydropyrido [2,3-d]pyrimidin-7(6H)-one hydrochloride

Tert-butyl 5-((R)-5-methyl-7-oxo-5,6,7,8-tetrahydropyrido[2,3-d]pyrimidin-4-yl)-2,5-diazabicyclo[4.1.0]he ptane-2-carboxylate (0.28 g) was dissolved in dichloromethane (5 mL) at 20°C, hydrogen chloride/1,4-dioxane (4.0 mL) was added, and the reaction solution reacted for 1 h. After the reaction was completed, the reaction solution was evaporated under reduced pressure to remove the solvent so as to yield a yellow solid (0.23 g) that was directly used at the next step.

c) Tert-butyl (2S)-2-(4-chlorophenyl)-3-(5-((R)-5-methyl-7-oxo-5,6,7,8-tetrahydropyrido[2,3-d]pyrimidin-4-y l)-2,5-diazabicyclo[4.1.0]heptan-2-yl)-3-oxopropyl)(isopropyl)carbamate

Under the protection of nitrogen gas, (5R)-4-(2,5-diazabicyclo[4.1.0]heptan-2-yl)-5-methyl-5,8-dihydropyridin[2,3-d]pyrimidin-7(6H) -one hydrochloride (0.20 g) and (S)-3-((tert-butoxycarbonyl)(isopropyl)amino)-2-(4-chlorophenyl)-propionic acid (0.22 g) were dissolved in N,N-dimethylformamide (5 mL) at 20°C, 2-(7-benzotriazole oxide)-N,N,N',N'-tetramethyluronium hexafluorophosphate (0.59 g) and 4-dimethylaminopyridine (0.48 g) were added, and the reaction solution reacted at 25°C for 4 h. After the reaction was completed, water (20 mL) was added to the reaction solution, the mixture was extracted with ethyl acetate (10 mL × 3), an obtained organic phase was washed with a saturated salt solution (10 mL × 2), and the solution was evaporated under reduced pressure to remove the organic phase and separated by column chromatography (dichloromethane: methanol=50: 1) to yield a yellow solid (0.18 g). LC-MS (ESI) m/z: 583 (M+H).

d) (R)-4-((1S,6R)-5-((S)-2-(4-chlorophenyl)-3-(isopropylamino)propionyl)-2,5-diazabicyclo[4.1.0] heptan-2-yl)-5-methyl-5,8-dihydropyrido[2,3-d]pyrimidin-7(6H)-one

Tert-butyl (2S)-2-(4-chlorophenyl)-3-(5-((R)-5-methyl-7-oxo-5,6,7,8-tetrahydropyrido[2,3-d]pyrimidin-4-y l)-2,5-diazabicyclo[4.1.0]heptan-2-yl)-3-oxopropyl)(isopropyl)carbamate (0.18 g) was dissolved in dichloromethane (2 mL) at 20°C, trifluoroacetic acid (0.86 mL) was added, and the reaction solution reacted for 3 h. After the reaction was completed, dichloromethane (10 mL) was added to the reaction solution, a 2 M sodium hydroxide solution was dropwise added at 0°C to regulate the pH of the mixture to 12, the solution was separated, an obtained organic phase was washed with a saturated salt solution (5 mL), and the solution was dried with anhydrous sodium sulfate and evaporated under reduced pressure to remove the organic phase so as to yield a yellow solid (0.10 g). The yellow solid was resolved by preparative high-performance liquid chromatography to yield isomer 1 (3 mg) and isomer 2 (12 mg). Preparative high-performance liquid chromatography conditions: chromatographic column: Aglient 5 μm prep-C18 50×21.2 mm; mobile phase A: water (containing 0.1 vol% of ammonium water (25-28 wt%)); and mobile phase B: methanol. Gradient: time: 0-10 min, 60-70% (volume percentage) of B phase.

Isomer 1: $RT_1$=5.3 min; LC-MS (ESI) m/z: 483 (M+H).

Isomer 2: RT=5.9 min; LC-MS (ESI) m/z: 483 (M+H); $^1$H NMR (400 MHz, $CDCl_3$) $\delta$ (ppm) 8.27 (d, J=7.6 Hz, 1H), 7.92 (s, 1H), 7.27-7.30 (m, 4H), 4.23-4.29 (m, 1H), 3.90-3.95 (m, 1H), 3.81-3.85 (m, 1H), 3.69-3.72 (m, 1H), 3.44-3.59 (m, 1H), 3.20-3.38 (m, 3H), 3.01-3.05 (m, 1H), 2.70-2.85 (m, 3H), 2.47-2.57 (m, 1H), 2.21-2.25 (m, 1H), 1.25-1.28 (m, 3H), 1.03-1.11 (m, 6H), 0.82-0.90 (m, 2H).

In the present application, configurations of the compounds of Example 1 were determined by single crystal diffraction, and it was determined that isomer 2 was the compound represented by formula 1 of the present application: Preparation of a single crystal: isomer 2 (30.0 mg) and isopropanol (2.0 mL) were placed in a 5 mL screw flask and stirred for 5 min until the solid was fully dissolved. Oxalic acid dihydrate (3.9 mg) was weighed and placed in the above flask, a white solid was gradually precipitated in the flask, the reaction solution was stirred at the room temperature for 3 h, and a large amount of white solid was precipitated in the flask. Methanol (1.0 mL) was placed in the flask, the white solid gradually disappeared, and after becoming clear, the solution was stirred for 1 h. The solution was filtered with a 0.22 μm microfiltration membrane to a 3 mL screw flask, and the opening of the flask was covered with a plastic wrap. The plastic warp covering the opening of the flask was pierced by a needle to form 8 small holes, the flask was placed at the room temperature for 7 d, and an oxalate single crystal of isomer 2 was obtained.

Single crystal diffraction experiment:

Single crystal X-ray diffractometer: BRUKER D8 VENTURE PHOTON II
Wavelength: Ga Kα (λ=1.34139 Å)
Test temperature: 190 K
Computer program for structural analysis: SHELXL-2018

Single crystal data: molecular formula: $C_{55}H_{72}Cl_2N_{12}O_9$; molecular weight: 1116.14; crystal system: hexagonal crystal system; space group: P61; unit cell parameters: a=25.8406(15) Å, b=25.8406(15) Å, c=45.916(3) Å, α=90°, β=90°, and γ=120°; unit cell volume: V=26552(4) $Å^3$; the number of molecular formulas contained in the unit cell: Z=12; calculated density: $D_{calc}$=0.838 g/$cm^3$; $R(F_o)$: 0.0730; $R_W(F_o^2)$: 0.2069; goodness of fit (S): 1.034; and Flack parameter: 0.066(9).

Structural description: single crystal X-ray diffraction and structural analysis show that the prepared single crystal is an oxalate isopropoxide of isomer 2. Asymmetric building blocks of the crystal include four isomer 2 molecules, two oxalic acid molecules, and two isopropanol molecules, and isomer 2 and oxalic acid form an oxalate. The single molecule of isomer 2 is shown in Fig. 1, and the asymmetric structural unit of the oxalate single crystal are shown in Fig. 2. The structural formula is shown below:

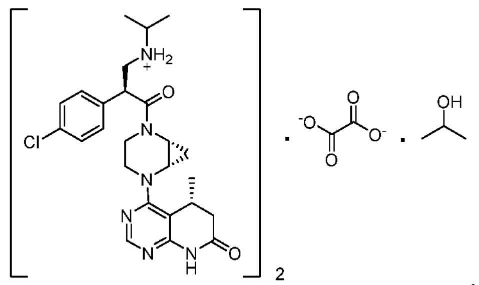

**Test Example 1 Test of AKT kinase inhibiting activity**

1. Materials and reagents

Envision model plate reader (Molecular Devices)
White 384-well plate (Thermo, Art. No. #264706)
Main reagents included in an HTRF kinEASE TK kit (Cisbio, Art. No. #62TKOPEC) TK-biotin substrate
Streptavidin-XL665
Europium-labeled tyrosine kinase substrate antibody

5× enzyme reaction buffer
SEB
HTRF assay buffer
AKT1 (Carna, Art. No. #01-101)
AKT2 (Carna, Art. No. #01-102)
AKT3 (Invitrogen, Art. No. #PV3185)
10 mM ATP (Invitrogen, Art. No. #PV3227)
1 M DTT (Sigma, Art. No. #D5545)
1 M $MgCl_2$ (Sigma, Art. No. #M8266)
Isomer 1 and isomer 2 of Example 1 of the present application
Positive control: GDC-0068

2. Experimental procedure

2.1 Preparation of reagents

Table 1 Concentrations of components of kinase reaction systems

| Reaction reagent | | AKT1 | AKT2 | AKT3 |
|---|---|---|---|---|
| Concentration of enzyme | Final concentration at the enzyme reaction step (10 μL) | 0.6 ng/ well | 0.1 ng/ well | 0.3 ng/ well |
| Concentration of ATP | | 2 μM | 20 μM | 10 nM |
| Concentration of TK-biotin substrate | | 2 μM | 2 μM | 2 μM |
| Enzyme reaction time | | 50 min | 50 min | 50 min |
| Concentration of streptavidin-XL665 | Final concentration in the overall reaction (20 μL) | 125 nM | 125 nM | 125 nM |
| Concentration of europium-labeled tyrosine kinase substrate antibody | | 1: 100 diluted | 1: 100 diluted | 1: 100 diluted |

1× kinase reaction buffer

A 1× kinase reaction buffer for 1 mL of kinase AKT1, AKT2 or AKT3 included 200 μL of 5× kinase reaction buffer, 5 μL of 1 M $MgCl_2$, 1 μL of 1 M DTT, and 794 μL of ultra-pure water.

5× TK-biotin substrate and ATP working solution

Specific concentrations of the TK-biotin substrate and ATP are shown in Table 1.
The substrate and ATP were respectively diluted with the 1× kinase reaction buffer to a concentration 5 times the reaction concentration.

5× kinase working solution

The concentration for enzyme screening is shown in Table 1. A 5× enzyme working solution was prepared from the 1× kinase reaction buffer.

4× streptavidin-XL665 working solution

The concentration of streptavidin-XL665 in the reaction is shown in Table 1. A 4× streptavidin-XL665 working solution was prepared from the assay buffer.

4× europium-labeled tyrosine kinase substrate antibody working solution

The europium-labeled tyrosine kinase substrate antibody was 100-fold diluted with the assay reaction buffer to obtain a working solution.

## 2.2 Experimental process

After all the reagents were prepared according to the above method, except for the enzyme, the reagents were equilibrated to the room temperature and loaded.

a) first, a compound stock solution (10 mM DMSO solution) was diluted with DMSO to obtain a 100 μM compound solution, the compound solution was diluted with the 1× kinase reaction buffer to obtain a 2.5 μM compound working solution (containing 2.5% DMSO). A 2.5% DMSO solution was prepared from the 1× kinase reaction buffer, and the 2.5 μM compound working solution was diluted 7 times with the 2.5% DMSO solution according to a 4-fold gradient to obtain compound working solutions at 8 concentrations (2500 nM, 625 nM, 156 nM, 39 nM, 9.8 nM, 2.4 nM, 0.6 nM, and 0.15 nM). Except for control wells, 4 μL of diluted compound working solution was placed in each reaction well, and 4 μL of previously prepared 2.5% DMSO/kinase buffer was placed in each control well.
b) 2 μL of previously prepared TK-biotin substrate solution (the concentration of the substrate for enzyme screening is shown in Table 1) was placed in each reaction well.
c) 2 μL of previously prepared enzyme solution (the concentration of the enzyme is shown in Table 1) was placed in each reaction well except for negative wells, and 2 μL of 1× kinase reaction buffer corresponding to the enzyme was placed in each negative well to make up the volume. The plate was sealed with a sealing film, and the reaction solution was mixed until uniform and incubated at the room temperature for 10 min to allow the compound to fully react with and bind to the enzyme.
d) 2 μL of ATP solution was placed in each reaction well to initiate a kinase reaction (the concentration of ATP for enzyme screening and reaction time are shown in Table 1).
e) 5 min before the kinase reaction was completed, an assay solution was prepared. Streptavidin-XL665 and a europium-labeled tyrosine kinase substrate antibody (1: 100) assay solution (the concentration of the assay reagent is shown in Table 1) were prepared from the assay buffer in the kit.
f) After the kinase reaction was completed, 5 μL of diluted streptavidin-XL665 was placed in each reaction well and mixed with the reaction solution until uniform, and the diluted europium-labeled tyrosine kinase substrate antibody assay solution was immediately added.
g) The plate was sealed, the reaction solution was mixed until uniform and reacted at the room temperature for 1 h, and fluorescence signals were detected by using an ENVISION (Perkinelmer) instrument (320 nm stimulation, 665 nm, 615 nm emission). An inhibition rate in each well was calculated from all active wells and background signal wells, a mean value of repetitive wells was calculated, and the half inhibitory activity (IC50) of each compound to be tested was fitted by using the professional drawing analysis software PRISM 6.0.

Table 2 Experimental loading process

| | Kinase reaction system | Control group | |
|---|---|---|---|
| Enzyme reaction step (10 μL) | Sample group | Negative control | Positive control |
| Isomer 1 or isomer 2 | 4 μL | 4 μL of 2.5% DMSO/kinase buffer | 4 μL of 2.5% DMSO/ kinase buffer |
| TK-biotin-labeled substrate | 2 μL | 2 μL | 2 μL |
| Kinase | 2 μL | 2 μL of kinase buffer | 2 μL |
| Seal with a film, and incubate at the room temperature for 10 min | | | |
| ATP | 2 μL | 2 μL | 2 μL |
| Seal with a film, and incubate at the room temperature for 50 min | | | |
| Detection steps (10 μL) | | | |
| Streptavidin-XL665 | 5 μL | 5 μL | 5 μL |
| Europium-labeled tyrosine kinase substrate antibody | 5 μL | 5 μL | 5 μL |
| Seal with a film, and incubate at the room temperature for 1 h | | | |
| Detection light: 320 nm, emitted light: 665 nm, 615 nm | | | |

**2.3 Data analysis**

$$ER = \text{fluorescence value at 665 nm} / \text{fluorescence value at 615 nm}$$

$$\text{Inhibition rate} = (ER_{\text{positive control}} - ER_{\text{sample}}) / (ER_{\text{positive control}} - ER_{\text{negative control}}) \times 100\%$$

**3. Experimental results**

Experimental results are shown in Table 3.

Table 3 AKT inhibiting activity

| Compound | Chemical structure | AKT1 enzyme | AKT2 enzyme | AKT3 enzyme |
|---|---|---|---|---|
| | | activity $IC_{50}$ (nM) | activity $IC_{50}$ (nM) | activity $IC_{50}$ (nM) |
| Isomer 1 of Example 1 | 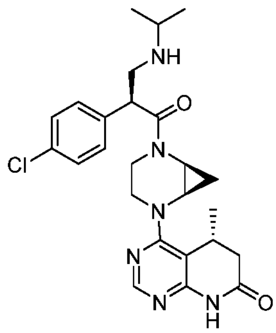 Isomer 1 | 62 | 542 | 13 |
| Isomer 2 of Example 1 | 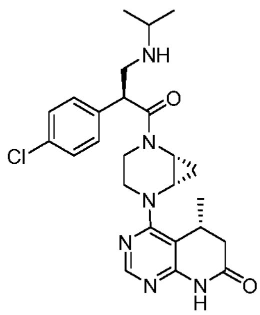 Isomer 2 | 0.35 | 6.3 | 0.09 |
| Positive control GDC-0068 | 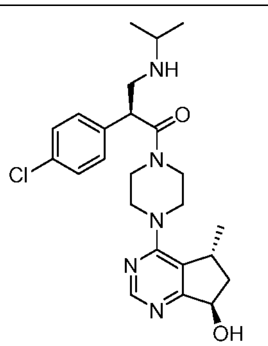 | 3.2 | 1.7 | 2.5 |

**Example 2 Preparation of salts of the compound represented by formula 1**

About 25 mg of compound represented by formula 1 and 1.05 equivalents of acid (for hydrochloric acid, a case of 2.10 equivalents was also set) were respectively added to 1 mL of solvent, and the reaction solution was stirred at the room temperature for 2 d. An obtained clear solution was attempted to crystallize by stirring at 5°C and slow evaporation, and a solid was separated by centrifugation, blast-dried or dried under reduced pressure at 40°C for 2-5 h, and characterized by XRPD and $^1$HNMR. Salinization results are shown in the table below. Salt form was determined by XRPD, and molar ratio of free base of the compound represented by formula 1 to acid radicals (i.e., a molar ratio of cations of the compound represented by formula 1 to acid radicals) was determined by $^1$HNMR.

Table 4 Salinization results of the compound represented by formula 1

| Acid | Solvent for a salt forming reaction* | Result | |
|---|---|---|---|
| | | Salt form | Salinization results (molar ratio of free alkali of the compound represented by formula 1 to acid radicals) |
| Sulfuric acid | Dichloromethane/n-heptane | Amorphous | Sulfate (1: 1) |
| Hydrobromic acid | Isopropanol/n-heptane; or acetone/n-heptane; or ethyl acetate/n-heptane; or toluene/n-heptane; or dichloromethane/n-heptane | | No salt is formed |
| Phosphoric acid | Isopropanol/n-heptane | Amorphous | Phosphate (1: 1) |
| Methanesulfonic acid | Ethyl acetate/n-heptane | Amorphous | Mesylate (1: 1) |
| Acetic acid | Isopropanol/n-heptane; or acetone/n-heptane; or ethyl acetate/n-heptane; or toluene/n-heptane; or dichloromethane/n-heptane | | No salt is formed |
| Isethionic acid | Ethyl acetate/n-heptane | Amorphous | Isethionate (1: 1) |
| α-naphthalenesulfonic acid | Ethyl acetate/n-heptane | Amorphous | α-naphthalenesulfonate (1: 1) |
| p-toluenesulfonic acid | Toluene/n-heptane | Amorphous | p-toluenesulfonate (1: 1) |
| 1,2-ethanedisulfonic acid | Isopropanol/n-heptane | Amorphous | 1,2-ethanedisulfonate (1: 1) |
| Oxalic acid | Toluene/n-heptane | Amorphous | Oxalate (1: 1) |
| Maleic acid | Isopropanol/n-heptane | Amorphous | Maleate (1: 1) |
| Fumaric acid | Isopropanol/n-heptane | Amorphous | Fumarate (1: 1) |
| Citric acid | Acetone/n-heptane | Amorphous | Citrate (1: 1) |
| Succinic acid | Isopropanol/n-heptane | Amorphous | Succinate |
| L-glutamic acid | Toluene/n-heptane | Amorphous | L-glutamate (1: 1) |
| L-tartaric acid | Isopropanol/n-heptane | Amorphous | L-tartrate (1: 1) |
| D-glucuronic acid | Toluene/n-heptane | Amorphous | D-glucuronate (1: 1) |
| Hippuric acid | Ethyl acetate/n-heptane | Mixture of amorphous and crystal | Hippurate (1: 1) |

(continued)

| Acid | Solvent for a salt forming reaction* | Result | |
|---|---|---|---|
| | | Salt form | Salinization results (molar ratio of free alkali of the compound represented by formula 1 to acid radicals) |
| L-ascorbic acid | Acetone/n-heptane | Mixture of amorphous and crystal | L-ascorbate (1: 1) |
| L-malic acid | Acetone/n-heptane | Amorphous | L-malate (1: 1) |
| Benzoic acid | Ethyl acetate/n-heptane | Amorphous | Benzoate (1: 1) |
| Gentisic acid | Isopropanol/n-heptane | Mixture of amorphous and crystal | Gentisate (1: 1) |
| Hydrochloric acid | Toluene/n-heptane | Amorphous | Hydrochloride (1: 2) |
| Hydrochloric acid | Ethyl acetate/n-heptane | Amorphous | Hydrochloride (1: 1) |
| *: in the solvents for salification reaction, a volume ratio of isopropanol, acetone, ethyl acetate, toluene and dichloromethane to n-heptane is 1: 2. | | | |

XRPD patterns of the sulfate, the phosphate, the isethionate, the α-naphthalenesulfonate, and the L-malate are respectively shown in Fig. 3 to Fig. 7.

**Example 3** Preparation of a monohydrochloride of the compound represented by formula 1

The compound represented by formula 1 (2 g) and toluene (10 mL) were placed in a 20 mL vial and shaken at the room temperature until the solid was fully dissolved. The clear solution was placed in a 100 mL double-layer glass jacketed reactor, a 4 mol/L hydrogen chloride-ethyl acetate solution (0.99 mL) was placed in the reactor, and the reaction solution was stirred for reaction for 15 min. N-heptane (40 mL) was placed in the reactor, and the reaction solution was stirred for curing at the room temperature for 2 h. After being cured, the reaction solution was subjected to suction filtration, and an obtained wet filter cake was dried in vacuum at 40°C for 19 h to yield a white solid powdery monohydrochloride of the compound represented by formula 1 (1.97 g).

$^1$HNMR (400 MHz, DMSO-$d_6$): 10.51 (s, 1H), 9.06 (s, 1H), 8.54 (s, 1H), 8.21 (s, 1H), 7.13-7.52 (m, 4H), 4.51-4.94 (m, 1H), 3.88-4.19 (m, 1H), 3.50-3.81 (m, 3H), 2.97-3.40 (m, 4H), 2.73-2.83 (m, 1H), 2.23-2.31 (m, 1H), 1.07-1.30 (m, 8H), 0.83-0.98 (m, 4H), 0.05 (q, J=5.2 Hz, 1H).

The XRPD pattern of the monohydrochloride of the compound represented by formula 1 is shown in Fig. 8.

**Example 4** Preparation of a dihydrochloride of the compound represented by formula 1

The compound represented by formula 1 (2 g) and toluene (10 mL) were placed in a 100 mL double-layer glass jacketed reactor and stirred at the room temperature until the solid was fully dissolved. A 4 mol/L hydrogen chloride-ethyl acetate solution (2.18 mL) was placed in the reactor, and the reaction solution was stirred for reaction for 15 min. N-heptane (40 mL) was placed in the reactor, and the reaction solution was stirred for curing at the room temperature for 4 h. After being cured, the reaction solution was subjected to suction filtration, and an obtained wet filter cake was dried in vacuum at 40°C for 6 h to yield a white solid powdery dihydrochloride of the compound represented by formula 1 (2.25 g).

$^1$HNMR (400 MHz, DMSO-$d_6$): 10.77 (s, 1H), 9.47 (s, 1H), 8.80 (s, 1H), 8.34 (s, 1H), 7.12-7.51 (m, 4H), 6.68 (s, 1H), 4.64-5.11 (m, 1H), 3.92-4.24 (m, 1H), 3.50-3.82 (m, 3H), 3.22-3.37 (m, 3H), 2.78-3.05 (m, 2H), 2.26-2.34 (m, 1H), 1.09-1.31 (m, 8H), 0.83-0.96 (m, 4H), 0.15 (q, J=5.2 Hz, 1H).

The XRPD pattern of the dihydrochloride of the compound represented by formula 1 is shown in Fig. 9.

**Example 5** Preparation of a fumarate of the compound represented by formula 1

The compound represented by formula 1 (25 mg) and isopropanol (1 mL) were placed in a 3 mL vial and

magnetically stirred at the room temperature until the solid was fully dissolved. Solid fumaric acid (6.31 mg) was placed in the 3 mL vial, and the reaction solution was magnetically stirred for reaction. After the reaction solution was stirred for 18 h, n-heptane (2 mL) was placed in the 3 mL vial, and the reaction solution was stirred for 18 h. The reaction solution was subjected to suction filtration, and an obtained wet filter cake was dried in vacuum at 40°C for 3 h to yield a white solid powdery fumarate of the compound represented by formula 1.

$^1$HNMR (400 MHz, DMSO-$d_6$): 10.49 (s, 1H), 8.20 (s, 1H), 7.34-7.48 (m, 4H), 6.52 (s, 2H), 4.37-4.76 (m, 1H), 3.88-4.18 (m, 1H), 3.70-3.81 (m, 2H), 3.34-3.54 (m, 2H), 3.03-3.21 (m, 4H), 2.90 (dd, J=11.6, 4.8 Hz, 1H), 2.76 (dd, J=16.4, 6.0 Hz, 1H), 2.22-2.30 (m, 1H), 1.04-1.32 (m, 8H), 0.85-0.93 (m, 4H), 0.08 (q, J=5.2 Hz, 1H).

The XRPD pattern of the fumarate of the compound represented by formula 1 is shown in Fig. 10.

In the present application, as demonstrated by Test Example 1 above, the compound represented by formula 1 of the present application has an inhibiting effect on the AKT kinase activity, and correspondingly, the pharmaceutically acceptable salt, such as a fumarate, a mesylate, an isethionate, an α-naphthalenesulfonate, a p-toluenesulfonate, a 1,2-ethanedisulphonate, an oxalate, a maleate, a citrate, a succinate, an L-(+)-tartrate, a hippurate, an L-ascorbate, an L-malate, a benzoate, a gentisate, a monohydrochloride, a dihydrochloride, a sulfate, and a phosphate, of the compound represented by formula 1 of the present application also has an inhibiting effect on the AKT kinase activity. Therefore, the pharmaceutically acceptable salt of the compound represented by formula 1 and the pharmaceutical composition comprising the salt of the present application can be used for preventing and/or treating an AKT protein kinase-mediated disease or disease state, and further can be used for preparing a medicament for preventing and/or treating an AKT protein kinase-mediated disease or disease state. Much further, compared with the compound represented by formula 1, the pharmaceutically acceptable salt of the compound represented by formula 1 of the present application has higher stability and better physical and chemical properties than the compound represented by formula 1, so it is more favorable for production and application.

The above are preferred embodiments of the present application only, but are not intended to limit the present application. Any modification, equivalent replacement, and improvement made within the spirit and principle of the present application shall fall within the protection scope of the present application.

## Claims

1. A pharmaceutically acceptable salt of a compound represented by formula 1, the salt selected from a salt of organic acid or a salt of inorganic acid, wherein the salt of organic acid is selected from a fumarate, a mesylate, an isethionate, an α-naphthalenesulfonate, a p-toluenesulfonate, a 1,2-ethanedisulphonate, an oxalate, a maleate, a citrate, a succinate, an L-(+)-tartrate, a hippurate, an L-ascorbate, an L-malate, a benzoate, or a gentisate, and the salt of inorganic acid is selected from a hydrochloride, a sulfate or a phosphate,

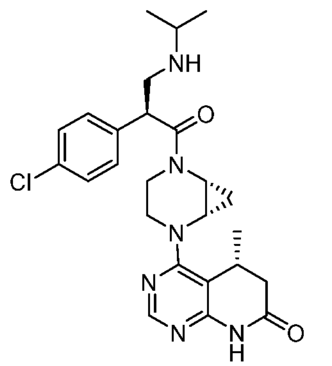

1

.

2. The salt according to claim 1, wherein the salt is selected from a fumarate and a hydrochloride.

3. The salt according to claim 1, wherein a ratio of the compound represented by formula 1 to organic acid is 1: 1.

4. The salt according to claim 1, wherein a ratio of the compound represented by formula 1 to hydrogen chloride is 1: 1 or 1: 2.

5. The salt according to claim 1, wherein a ratio of the compound represented by formula 1 to hydrogen chloride is 1: 2.

**6.** The salt according to claim 1, wherein the salt is a fumarate, and a molar ratio of the compound represented by formula 1 to fumaric acid is 1: 1.

**7.** A preparation method of the pharmaceutically acceptable salt of the compound represented by formula 1 according to any one of claims 1 to 6, comprising a step of salification reaction of the compound represented by formula 1 with the corresponding acid.

**8.** The method according to claim 7, wherein a solvent for salification reaction is selected from a mixed solvent of an alcohol solvent and an alkane solvent, a mixed solvent of a ketone solvent and an alkane solvent, a mixed solvent of an ester solvent and an alkane solvent, a mixed solvent of a benzene solvent and an alkane solvent, and a mixed solvent of a halogenated hydrocarbon solvent and an alkane solvent.

**9.** A pharmaceutical composition comprising the salt according to any one of claims 1 to 6.

**10.** The pharmaceutically acceptable salt of the compound represented by formula 1 according to any one of claims 1 to 6 or the pharmaceutical composition according to claim 9 for use as a medicament.

**11.** Use of the salt according to any one of claims 1 to 6 or the pharmaceutical composition according to claim 9 in the prevention and/or treatment of an AKT protein kinase-mediated disease or disease state.

**12.** Use of the salt according to any one of claims 1 to 6 or the pharmaceutical composition according to claim 9 in the preparation of a medicament for preventing and/or treating an AKT protein kinase-mediated disease or disease state.

**13.** The use according to claim 11 or 12, wherein the AKT protein kinase-mediated disease or disease state is cancer, preferably breast cancer, prostate cancer or ovarian cancer, and more preferably prostate cancer.

**14.** A method for preventing and/or treating an AKT protein kinase-mediated disease or disease state, comprising a step of administering the salt according to any one of claims 1 to 6 or the pharmaceutical composition according to claim 9 to the subject in need.

**15.** The method according to claim 14, wherein the AKT protein kinase-mediated disease or disease state is cancer, preferably breast cancer, prostate cancer or ovarian cancer, and more preferably prostate cancer.

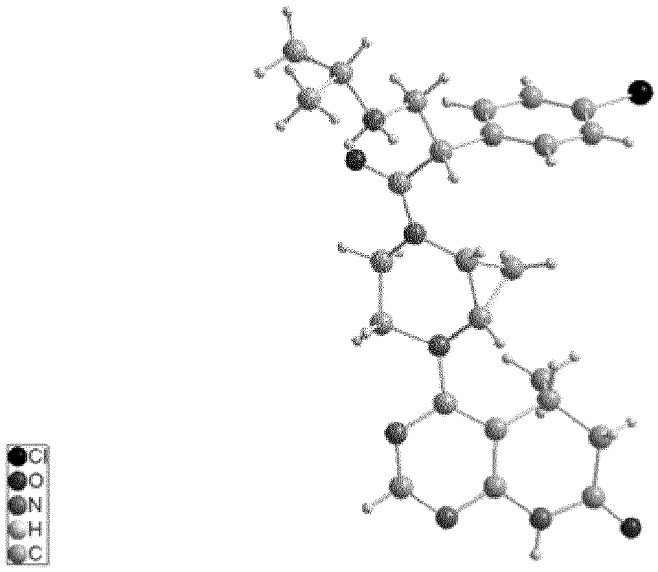

Fig. 1

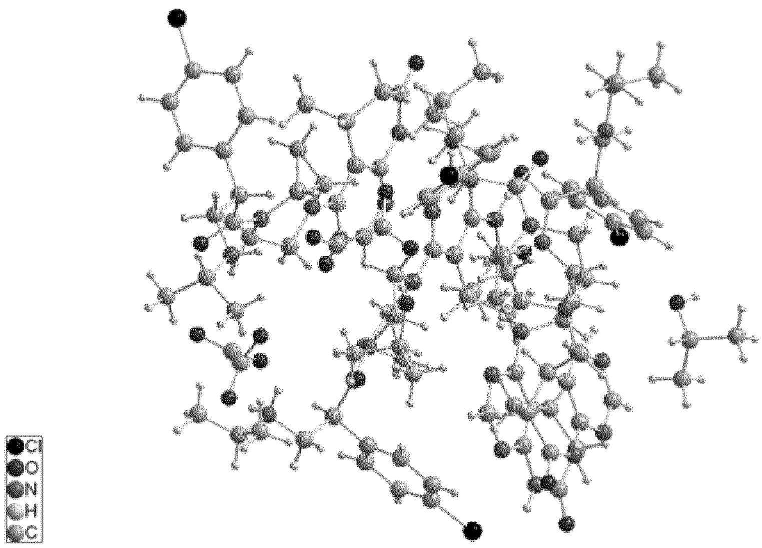

Fig. 2

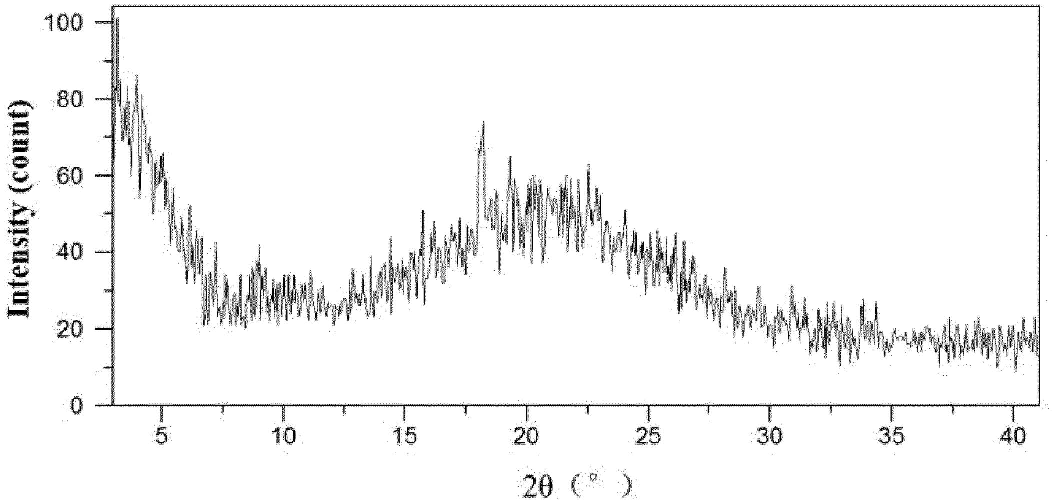


Fig. 3

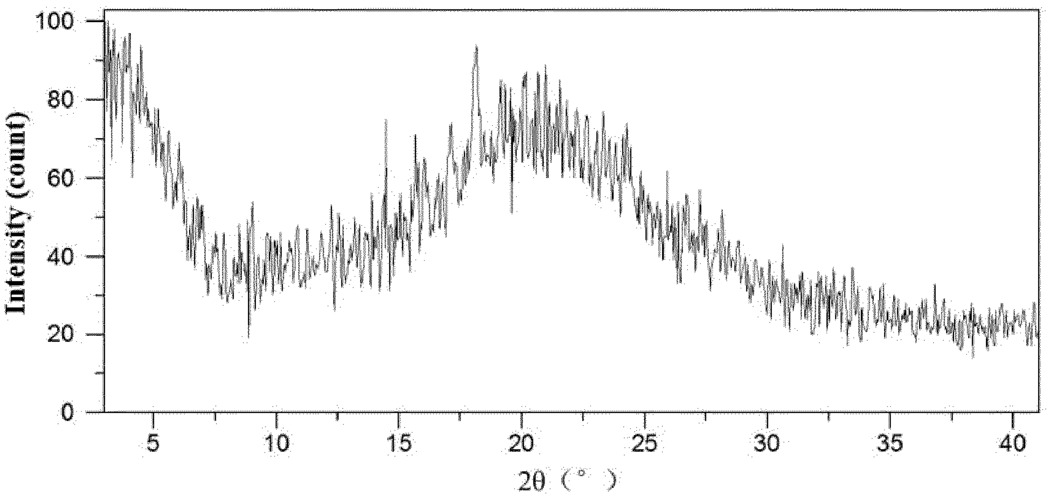


Fig. 4

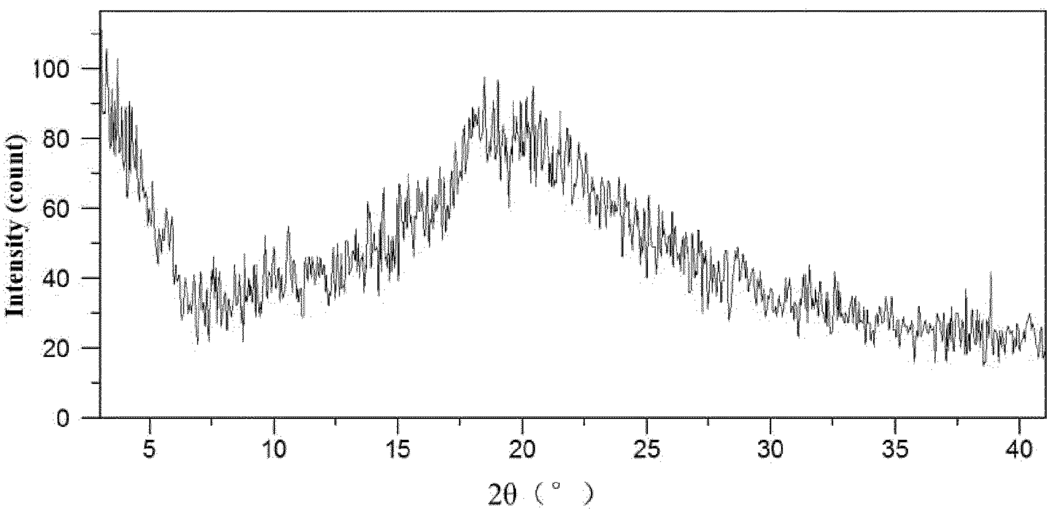


Fig. 5

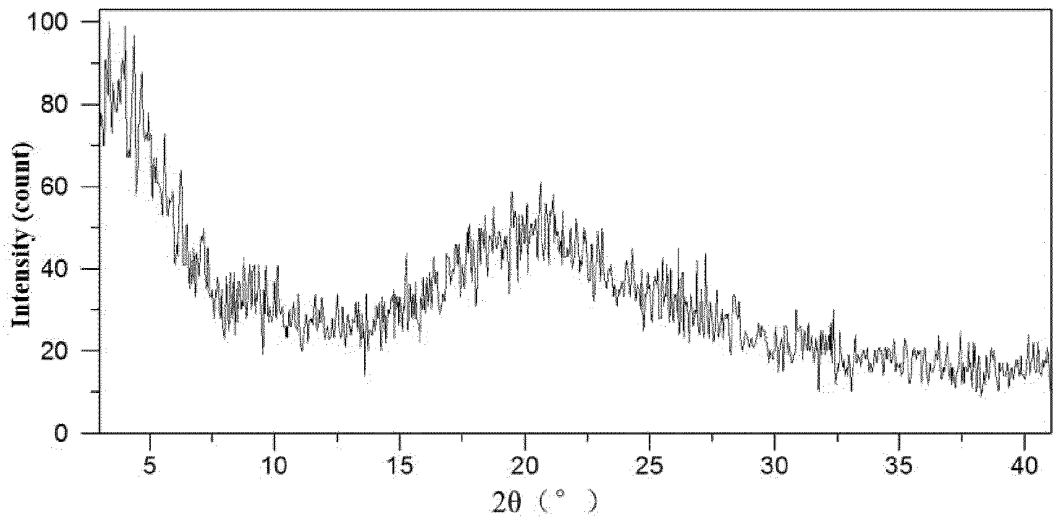


Fig. 6

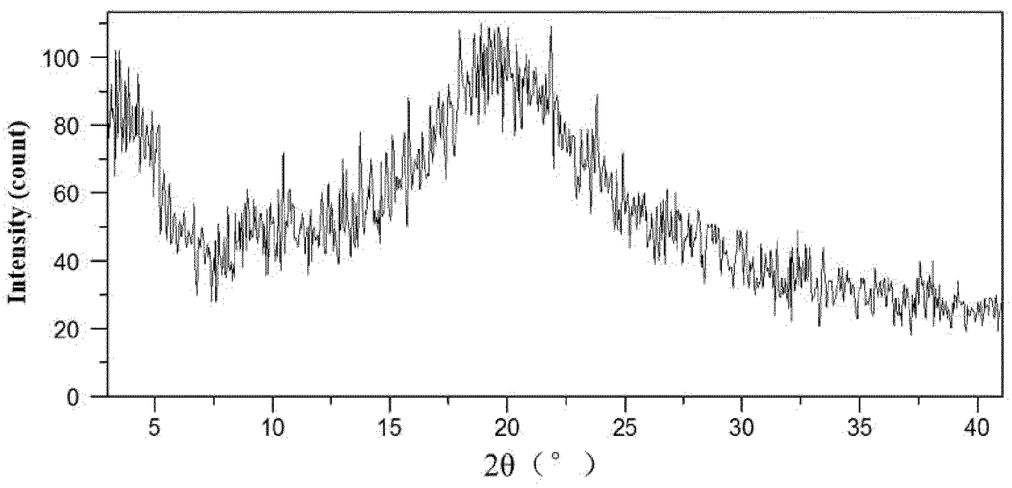


Fig. 7

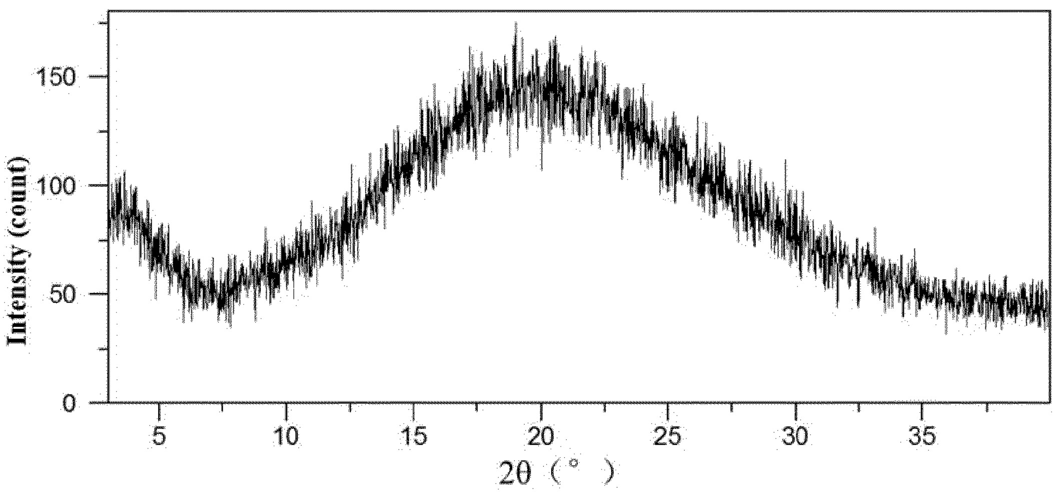


Fig. 8

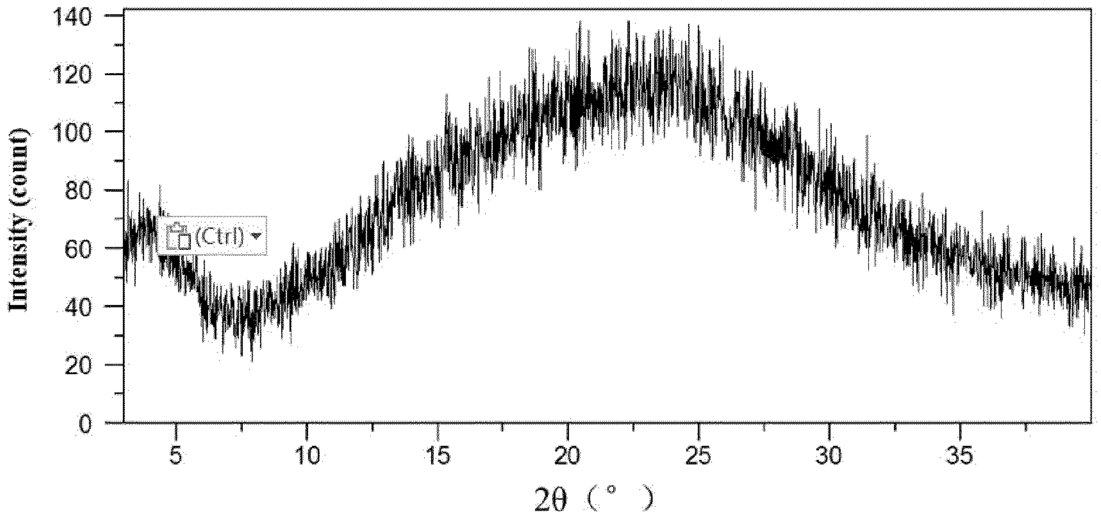
Intensity (count)
140
120
100
80
60
40
20
0
(Ctrl)
5
10
15
20
25
30
35
2θ（°）

Fig. 9

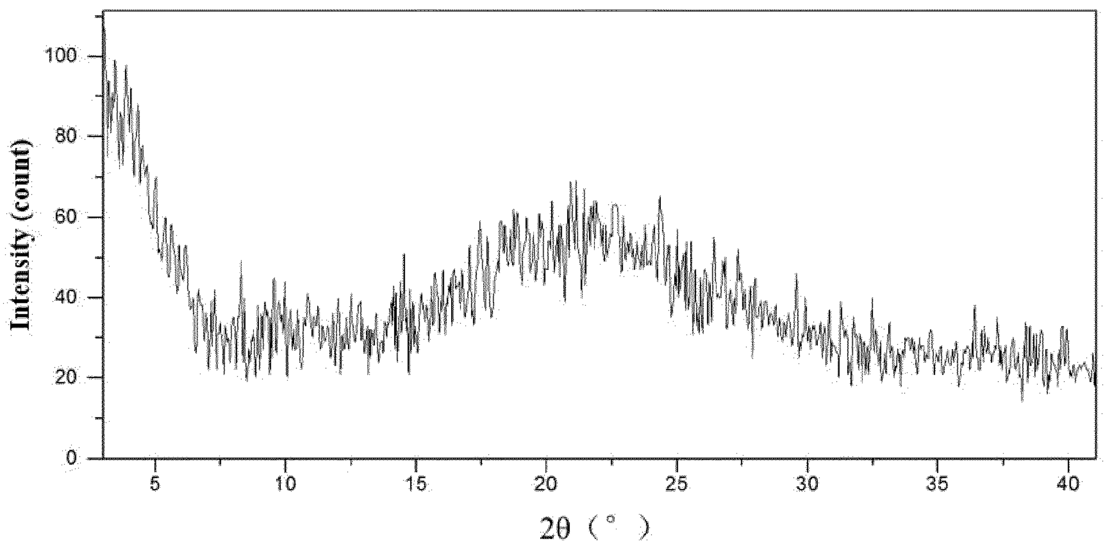
Intensity (count)
100
80
60
40
20
0
5
10
15
20
25
30
35
40
2θ（°）

Fig. 10

## INTERNATIONAL SEARCH REPORT

International application No.
**PCT/CN2021/107815**

**A. CLASSIFICATION OF SUBJECT MATTER**

C07D 471/04(2006.01)i； A61K 31/519(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, VEN, WOTXT, EPTXT, USTXT, CNKI, 万方, caplus, marpat, registry: 正大天晴, 马昌友, 田禾, 赵建良, 陈东晖, 吴舰, 徐丹, 朱春霞, 田舟山, 吡啶, 嘧啶酮, 激酶, 癌症, 肿瘤, 蛋白激酶B, AKT, PKB, kinase?, cancer?, tumor?, tumour?, protein kinase B, +pyridin+, +pyramid+

**C. DOCUMENTS CONSIDERED TO BE RELEVANT**

| Category* | Citation of document, with indication, where appropriate, of the relevant passages | Relevant to claim No. |
|---|---|---|
| PX | WO 2020156437 A1 (NANJING CHIA TAI TIANQING PHARMACEUTICAL CO., LTD.) 06 August 2020 (2020-08-06)<br>description page 14, embodiment 15, experiment 1 | 1-10, 12, 13 (in part) |
| A | CN 1882347 A (ARRAY BIOPHARMA, INC.) 20 December 2006 (2006-12-20)<br>entire document, in particular description page 11 line 27 to page 20 line 12, page 30 line 15 to page 33 line 27 | 1-10, 12, 13 (in part) |
| A | CN 102574852 A (ELI LILLY AND COMPANY) 11 July 2012 (2012-07-11)<br>entire document | 1-10, 12, 13 (in part) |

☐ Further documents are listed in the continuation of Box C.　　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 August 2021** | **27 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/107815**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11,14-15,13(部分)**
because they relate to subject matter not required to be searched by this Authority, namely:

[1] The technical solution in claims 11, 14-15, and the technical solution in claim 13 referring to claim 11 relate to methods for treatment of the human body or animal body, and therefore relate to a subject matter under PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/107815**

| Patent document cited in search report | Publication date (day/month/year) | Patent family member(s) | Publication date (day/month/year) |
|---|---|---|---|
| WO 2020156437 A1 | 06 August 2020 | CN 113272304 A | 17 August 2021 |
| CN 1882347 A | 20 December 2006 | NO 20062884 L | 18 August 2006 |
| | | JP 2007512364 A | 17 May 2007 |
| | | IL 175221 D0 | 31 October 2006 |
| | | US 2016152576 A9 | 02 June 2016 |
| | | NZ 590160 A | 27 July 2012 |
| | | US 2010168123 A1 | 01 July 2010 |
| | | RU 2006121990 A | 27 December 2007 |
| | | AU 2004293026 A1 | 09 June 2005 |
| | | NO 20062884 A | 18 August 2006 |
| | | IL 208520 D0 | 30 December 2010 |
| | | US 2005130954 A1 | 16 June 2005 |
| | | US 2014148436 A1 | 29 May 2014 |
| | | KR 20070026332 A | 08 March 2007 |
| | | CN 102786482 A | 21 November 2012 |
| | | US 8680114 B2 | 25 March 2014 |
| | | IL 175221 A | 31 May 2015 |
| | | CA 2546754 A1 | 09 June 2005 |
| | | NZ 547327 A | 28 August 2009 |
| | | KR 101223914 B1 | 18 January 2013 |
| | | AU 2004293026 B2 | 19 January 2012 |
| | | EP 1684694 A2 | 02 August 2006 |
| | | KR 20110137838 A | 23 December 2011 |
| | | ZA 200604069 B | 29 April 2009 |
| | | BR PI0416852 A | 27 February 2007 |
| | | WO 2005051304 A2 | 09 June 2005 |
| | | ZA 200604069 A | 29 April 2009 |
| CN 102574852 A | 11 July 2012 | EA 201270590 A1 | 28 September 2012 |
| | | JP 2013508382 A | 07 March 2013 |
| | | US 8436002 B2 | 07 May 2013 |
| | | CA 2778291 A1 | 28 April 2011 |
| | | EA 020151 B1 | 30 September 2014 |
| | | CA 2778291 C | 11 February 2014 |
| | | KR 20120068941 A | 27 June 2012 |
| | | EP 2491032 B1 | 16 April 2014 |
| | | AU 2010310786 A1 | 19 April 2012 |
| | | WO 2011050016 A1 | 28 April 2011 |
| | | US 2012149684 A1 | 14 June 2012 |
| | | CN 102574852 B | 25 June 2014 |
| | | JP 5581390 B2 | 27 August 2014 |
| | | AU 2010310786 B2 | 27 March 2014 |
| | | MX 2012004780 A | 23 August 2012 |
| | | KR 101398268 B1 | 23 May 2014 |
| | | ES 2465094 T3 | 05 June 2014 |
| | | EP 2491032 A1 | 29 August 2012 |
| | | BR 112012011328 A2 | 22 November 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101631778 A
- CN 101578273 A
- CN 101511842 A
- CN 101970415 A